# EUROPEAN PATENT APPLICATION

(11) **EP 3 456 240 A1**
(43) Date of publication of application: **20.03.2019**
(21) Application number: 17796180.2
(22) Date of filing: 10.05.2017
(51) Int. Cl.: A61B 1/24, A61B 1/045

(54) **GINGIVITIS DIAGNOSIS ASSISTANCE DEVICE AND GINGIVITIS DIAGNOSIS ASSISTANCE SYSTEM**

(30) Priority: 10.05.2016 JP 2016094486; 23.02.2017 JP 2017031801
(71) Applicant: Kambara, Masaki, Osaka-shi, Osaka 558-0054 (JP)
(72) Inventor: Kambara, Masaki, Osaka-shi, Osaka 558-0054 (JP)
(74) Representative: Abel & Imray
(86) International application number: PCT/JP2017/017678
(87) International publication number: WO 2017/195820

(57) **Abstract**

A gingivitis diagnosis assistance device (20A) is provided with a cross-section data extraction unit (21) for extracting cross-section data in a depth direction of a gingivitis peripheral part from three-dimensional point group data of a mouth front, and a gingivitis diagnosis data generating unit (25) for processing the cross-section data into a format suitable for gingivitis diagnosis and generating gingivitis diagnosis data, the gingivitis diagnosis data generating unit (25) generating, as the gingivitis diagnosis data, a two-dimensional graph in which the left-right direction or the up-down direction of the mouth front is the first axis and the depth direction is the second axis, for example.

## Description

### Technical Field

The present invention relates to a gum diagnosis assisting apparatus and a gum diagnosis assisting system which assist dentists to make a gum diagnosis.

### Background Art

For early detection and/or prevention of odontopathies, it is necessary to be able to accurately and objectively diagnose a period from a healthy state to a development of diseases. However, since a dental preventive diagnosis largely depends on subjective experiences and intuitions of a dentist, diagnostic decisions may vary depending on dentists, and/or it is not possible to obtain certain objective data. To solve such problems, in particular, in relation to a diagnosis of a periodontic disease, a periodontic disease diagnosis assisting apparatus which improves the objectivity of measured data while the diagnostic criteria and the safety of the diagnosis are improved is publicly known (for example, see Patent Literature 1).

### Citation List

### Patent Literature

Patent Literature 1: JP 2015-116303 A

### Summary of Invention

### Technical Problem

Gingivitis and the like of the odontopathies are subjectively diagnosed by a dentist based on whether or not five indications (flare, swelling, pyrexia, bleeding, and morphological abnormality) of inflammation develop. The technique disclosed in Patent Literature 1 is specialized in a diagnosis of a periodontic disease and is difficult to be applied to a gum diagnosis. Thus, the gum diagnosis currently still needs to be based on visual observation, experiences, intuitions, and the like of a dentist.

In view of the foregoing, it is an object of the present invention to provide a gum diagnosis assisting apparatus and a gum diagnosis assisting system which assist a dentist to objectively make a gum diagnosis.

### Solution to Problem

A gum diagnosis assisting apparatus according to an aspect of the present invention includes a sectional data extractor configured to extract sectional data in a depth direction of a gum marginal portion from three-dimensional point group data of a frontal view of an oral cavity, and a gum diagnosis data generator configured to process the sectional data into a format suitable to a gum diagnosis to generate gum diagnosis data.

The gum diagnosis data generator may be configured to generate a two-dimensional graph as the gum diagnosis data, where a right-left direction of the frontal view of the oral cavity corresponds to a first axis and a depth direction of the frontal view of the oral cavity corresponds to a second axis.

The gum diagnosis data generator may be configured to generate a two-dimensional graph as the gum diagnosis data, where an up-down direction of the frontal view of the oral cavity corresponds to a first axis and a depth direction of the frontal view of the oral cavity corresponds to a second axis.

A gum diagnosis assisting apparatus of another aspect of the present invention includes a contour extractor configured to extract a contour of a gum marginal portion from a front photographic image of a tooth and gum to generate contour point group data, a curve fitting unit configured to perform function fitting on point group data of a border between the tooth and the gum of the contour point group data to derive a fitted curve, and a gum diagnosis data generator configured to generate gum diagnosis data by superimposing the fitted curve on the point group data of the border between the tooth and the gum.

The gum diagnosis assisting apparatus may further include a correlation calculator configured to calculate an index denoting a degree of correlation of the point group data of the border between the tooth and the gum with the fitted curve.

A gum diagnosis assisting system according to the present invention includes the gum diagnosis assisting apparatus, an imaging apparatus configured to generate original image data of a process target by the gum diagnosis assisting apparatus, and a display apparatus configured to display gum diagnosis data generated by the gum diagnosis assisting apparatus.

### Advantageous Effects of Invention

According to the present invention, gum diagnosis data is generated from three-dimensional point group data of a frontal view of an oral cavity and a front photographic image of a tooth and gum of a patient, and a dentist can make a gum diagnosis with reference to the gum diagnosis data. Thus, quantification and imaging enables a more reliable and objective gum diagnosis than a visual diagnosis. Moreover, it becomes possible to store the gum diagnosis data as digital data to track aging of the gum of the patient. This can improve the quality of a preventive diagnosis.

### Brief Description of Drawings

FIG. 1 is a view illustrating a configuration of a gum diagnosis assisting system according to a first embodiment.
FIG. 2A is a two-dimensional image of a frontal view of an oral cavity according to an example.
FIG. 2B is a view illustrating a three-dimensional point group data of the frontal view of the oral cavity corresponding to FIG. 2A.
FIG. 2C is a view illustrating an example of gum diagnosis data obtained from the three-dimensional point group data of FIG. 2B.
FIG. 2D is a view illustrating an example of gum diagnosis data obtained from the three-dimensional point group data of FIG. 2B.
FIG. 3 is a view illustrating a configuration of a gum diagnosis assisting system according to a second embodiment.
FIG. 4A is a front photographic image of a tooth and gum according to an example.
FIG. 4B is a view illustrating contour point group data obtained from the photographic image of FIG. 4A.
FIG. 4C is a view illustrating gum diagnosis data obtained by superimposing a fitted curve on the point group data of a border between the tooth and gum of FIG. 4B.
FIG. 5A is a front photographic image of a tooth and gum according to another example.
FIG. 5B is a view illustrating contour point group data obtained from the photographic image of FIG. 5A.
FIG. 5C is a view illustrating gum diagnosis data obtained by superimposing a fitted curve on the point group data of a border between the tooth and the gum of FIG. 5B.
FIG. 6A is a front photographic image of a tooth and gum according to still another example.
FIG. 6B is a view illustrating contour point group data obtained from the photographic image of FIG. 6A.
FIG. 6C is a view illustrating gum diagnosis data obtained by superimposing a fitted curve on the point group data of a border between the tooth and the gum of FIG. 6B.

### Description of Embodiments

With reference to the drawings as necessary, embodiments will be described in detail below. Note that unnecessarily detailed description may be omitted. For example, detailed description of already well-known matters and repeated description of substantially the same configurations may be omitted. This is to avoid that the following description becomes unnecessarily redundant and to facilitate understanding of the following description by those skilled in the art.

Note that the inventor provides the attached drawings and the following description for a full understanding of the present invention by those skilled in the art, but the inventor does not intend to limit the subject-matter in the claims by the attached drawings and the following description.

### First Embodiment

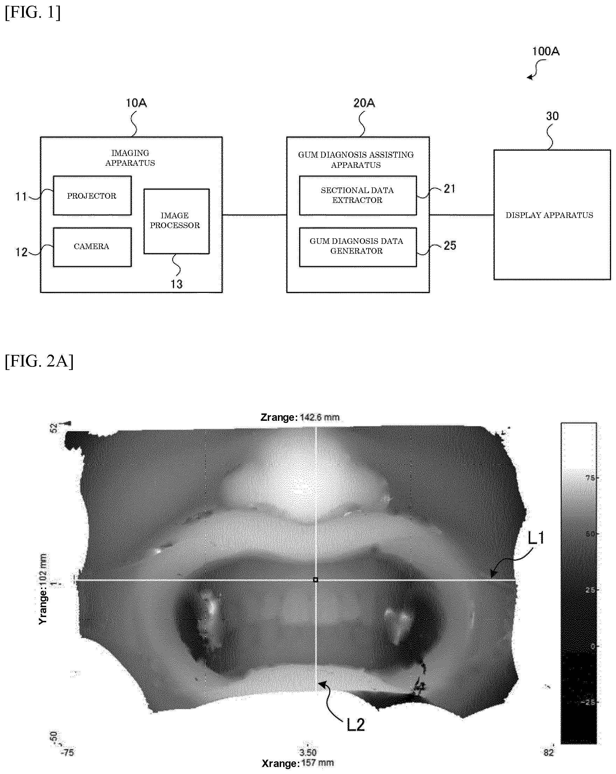

FIG. 1 is a view illustrating a configuration of a gum diagnosis assisting system according to a first embodiment. A gum diagnosis assisting system 100A according to the present embodiment includes an imaging apparatus 10A, a gum diagnosis assisting apparatus 20A, and a display apparatus 30.

The imaging apparatus 10A is an apparatus configured to generate three-dimensional point group data of a surface of an object. As the imaging apparatus 10A, for example, a three-dimensional measurement apparatus based on a phase shift method may be used.

Specifically, the imaging apparatus 10A includes a projector 11, a camera 12, and an image processor 13.

The projector 11 projects sinusoidal stripe-pattern light onto the surface of the object with the phase of the sinusoidal stripe-pattern light being shifted. The camera 12 captures reflection light from the surface of the object to generate an image. The image processor 13 analyzes the image captured with the phase of the stripe-pattern light being shifted, and from the phase difference, the image processor 13 acquires a three-dimensional shape (three-dimensional point group data) of the surface of the object. Such a phase shift method enables three-dimensional measurement with an accuracy of about several tens of micrometers in the depth direction in a relatively short time.

Note that the imaging apparatus 10A may use a spatial code method, an optical cutting method, or the like other than the phase shift method. Alternatively, a stereo camera is used as the camera 12, and three-dimensional measurement based on triangulation may be performed.

In the gum diagnosis assisting system 100A, the imaging apparatus 10A is used to acquire three-dimensional point group data of the frontal view of an oral cavity of a patient.

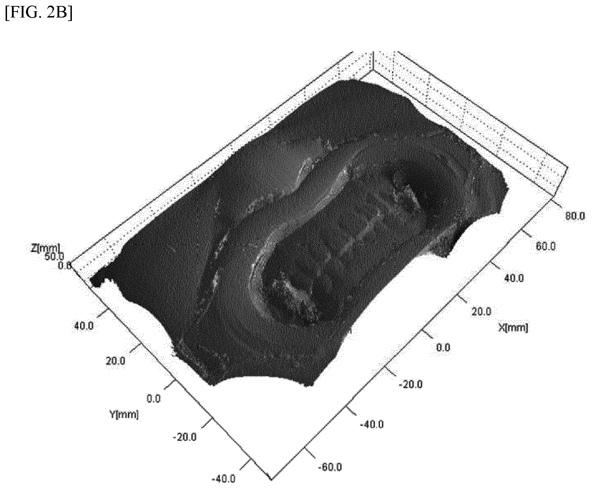

FIG. 2A shows an example of a two-dimensional image of the frontal view of the oral cavity imaged by the imaging apparatus 10A. FIG. 2B is a view illustrating a three-dimensional point group data of the frontal view of the oral cavity corresponding to FIG. 2A.

As illustrated in FIGS. 2A and 2B, the imaging apparatus 10A acquires the three-dimensional point group data of the frontal view of the oral cavity in a range of 157 mm in the right-left direction (X range), 102 mm in the up-down direction (Y range), and 142.6 mm in the depth direction (Z range).

Referring back to FIG. 1, the gum diagnosis assisting apparatus 20A is an apparatus configured to receive the three-dimensional point group data of the frontal view of the oral cavity from the imaging apparatus 10A and to process the three-dimensional point group data, thereby generating gum diagnosis data which helps a dentist make a gum diagnosis. The gum diagnosis assisting apparatus 20A may include a Personal Computer (PC), a mainframe computer such as a server apparatus, or the like. Alternatively, the gum diagnosis assisting apparatus 20A may be realized as Software as a Service (Saas) on the cloud. In this case, the imaging apparatus 10A, the gum diagnosis assisting apparatus 20A, and the display apparatus 30 are connected to one another via a network such as the Internet.

Specifically, the gum diagnosis assisting apparatus 20A includes a sectional data extractor 21 and a gum diagnosis data generator 25. Note that the sectional data extractor 21 and the gum diagnosis data generator 25 may include software, hardware, or a combination thereof.

The sectional data extractor 21 extracts sectional data in a depth direction of a gum marginal portion from the three-dimensional point group data of the frontal view of the oral cavity. That is, the sectional data extractor 21 extracts, from the three-dimensional point group data of the frontal view of the oral cavity, point group data of the XZ plane at an arbitrary location in the Y range and/or point group data of the YZ plane at an arbitrary location in the X range.

The gum diagnosis data generator 25 processes the sectional data in the depth direction of the gum marginal portion into a format suitable to the gum diagnosis to generate the gum diagnosis data. For example, the gum diagnosis data generator 25 generates, as the gum diagnosis data, a two-dimensional graph, where the right-left direction of the frontal view of the oral cavity corresponds to a horizontal axis and the depth direction of the frontal view of the oral cavity corresponds to the vertical axis, and/or a two-dimensional graph, where the up-down direction of the frontal view of the oral cavity corresponds to a horizontal axis and the depth direction of the frontal view of the oral cavity corresponds to the vertical axis.

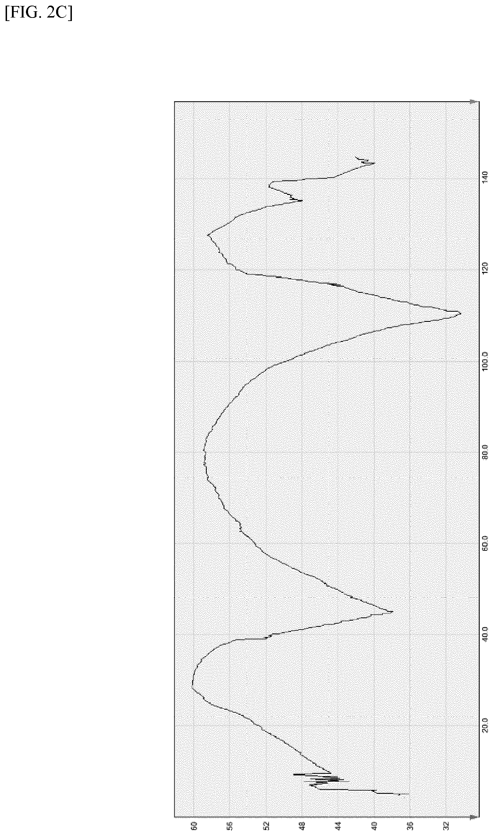

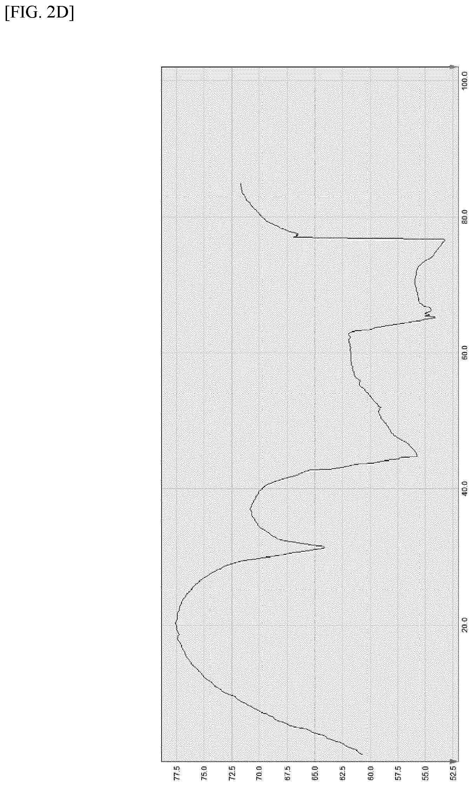

FIGS. 2C and 2D are views illustrating examples of the gum diagnosis data obtained from the three-dimensional point group data in FIG. 2B. The gum diagnosis data in FIG. 2C is a two-dimensional graph, where the right-left direction of the frontal view of the oral cavity corresponds to the horizontal axis and the depth direction of the frontal view of the oral cavity corresponds to the vertical axis, and the gum diagnosis data in FIG. 2C corresponds to the point group data of the XZ plane on a line L1 shown in FIG. 2A. The line L1 is a line denoting the arbitrary location in the Y range in the three-dimensional point group data of the frontal view of the oral cavity. The gum diagnosis data in FIG. 2D is a two-dimensional graph, where the up-down direction of the frontal view of the oral cavity corresponds to the horizontal axis and the depth direction of the frontal view of the oral cavity corresponds to the vertical axis, and the gum diagnosis data in FIG. 2D corresponds to the point group data of the YZ plane on a line L2 shown in FIG. 2A. In the two-dimensional image of the frontal view of the oral cavity of FIG. 2A, a portion protruding in a direction facing the viewer of the figure is represented by a large value along the vertical axis in the two-dimensional graphs in FIGS. 2C and 2D. That is, in the two-dimensional graphs, a swelling of the gum marginal portion is shown by a large value along the vertical axis.

Referring back to FIG. 1, the display apparatus 30 is an apparatus configured to display the gum diagnosis data generated by the gum diagnosis assisting apparatus 20A. For example, the display apparatus 30 displays the two-dimensional image of the frontal view of the oral cavity of FIG. 2A and the gum diagnosis data of FIG. 2C and/or FIG. 2D side by side. A dentist watches the images displayed on the display apparatus 30, which enables the dentist to objectively determine the degree of the swelling of the gum marginal portion.

### Second Embodiment

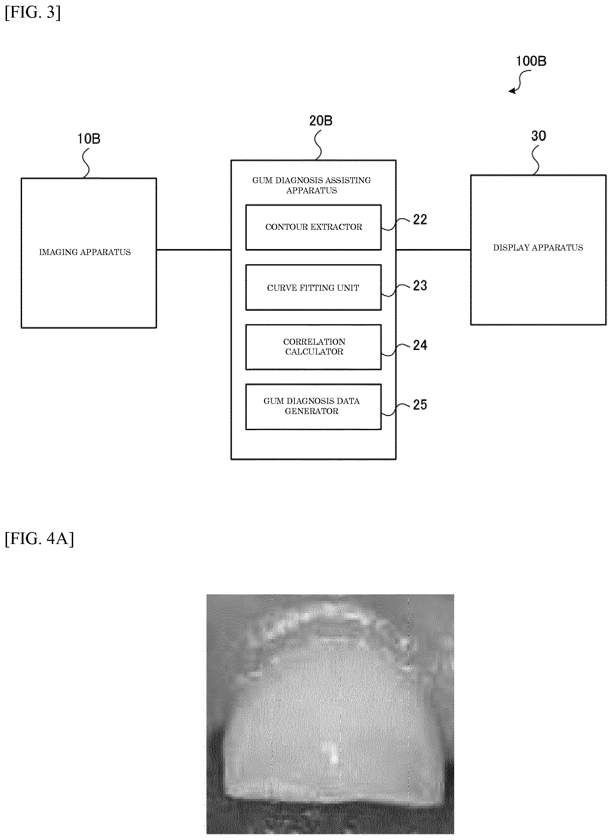

FIG. 3 is a view illustrating a configuration of a gum diagnosis assisting system according to a second embodiment. A gum diagnosis assisting system 100B according to the present embodiment includes an imaging apparatus 10B, a gum diagnosis assisting apparatus 20B, and a display apparatus 30.

The imaging apparatus 10B is an apparatus configured to image a still image of an object. As the imaging apparatus 10B, a general digital still camera can be used.

In the gum diagnosis assisting system 100B, the imaging apparatus 10B is used to acquire a front photographic image of a tooth and gum of a patient. FIGS. 4A, 5A, and 6A show various examples of the front photographic image of the tooth and the gum. Note that photographic images imaged by the imaging apparatus 10B may be in color or in grayscale.

Referring back to FIG. 3, the gum diagnosis assisting apparatus 20B is an apparatus configured to receive three-dimensional point group data of a frontal view of the oral cavity from the imaging apparatus 10B and to process the three-dimensional point group data, thereby generating gum diagnosis data which helps a dentist make the gum diagnosis. The gum diagnosis assisting apparatus 20B may include a PC, a mainframe computer such as a server apparatus, or the like. Alternatively, the gum diagnosis assisting apparatus 20B may be realized as Software as a Saas on the cloud. In this case, the imaging apparatus 10B, the gum diagnosis assisting apparatus 20B, and the display apparatus 30 are connected to one another via a network such as the Internet.

Specifically, the gum diagnosis assisting apparatus 20B includes a contour extractor 22, a curve fitting unit 23, a sectional data extractor 24, and a gum diagnosis data generator 25. Note that the contour extractor 22, the curve fitting unit 23, the sectional data extractor 24 and the gum diagnosis data generator 25 may include software, hardware, or a combination thereof.

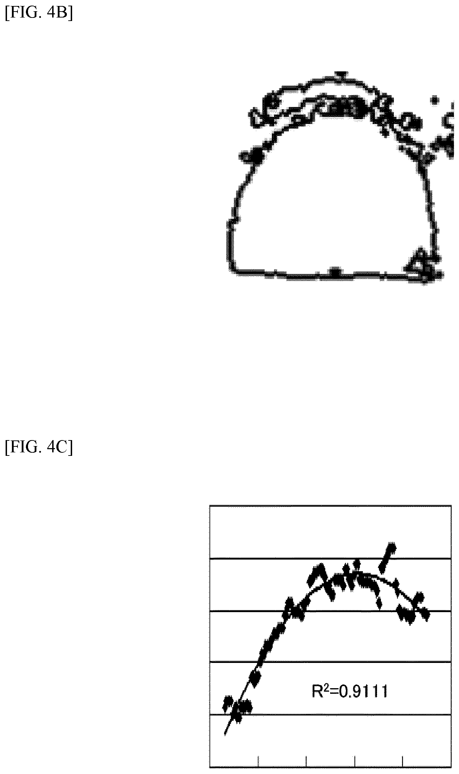

The contour extractor 22 extracts the contour of a gum marginal portion from the front photographic image of the tooth and the gum to generate contour point group data. Specifically, the contour extractor 22 calculates the difference of the luminance or the contrast between adjacent pixels in the photographic image to extract, as a contour, a part where the difference is large. FIGS. 4B, 5B, and 6B are views illustrating examples of the contour point group data obtained from the photographic image respectively of FIGS. 4A, 5A, and 6A. As can be seen from FIGS. 4B, FIG. 5B, and FIG. 6B, the contour of the gum marginal portion is extracted from the front photographic image of the tooth and the gum, so that the border between the tooth and the gum is shown as the contour point group data.

Referring back to FIG. 3, the curve fitting unit 23 performs function fitting on point group data of the border between the tooth and the gum of the contour point group data generated by the contour extractor 22 to derive a fitted curve. Here, as the fitted curve, a polynomial of an arbitrary order may be used. This is because the fitted curve of the border between the tooth and the gum has an individual difference, a personal difference, a secular difference, and the like. Thus, in the function fitting, polynomial of the fitted curve is not limited, and fitted curve which can approximate the border between the tooth and the gum with a smaller variation is found.

The correlation calculator 24 calculates an index denoting the degree of correlation of the fitted curve with the point group data of the border between the tooth and the gum. As the index, a correlation coefficient R, a determination coefficient R^2, or the like may be used.

The gum diagnosis data generator 25 generates the gum diagnosis data by superimposing the fitted curve on the point group data of the border between the tooth and the gum.

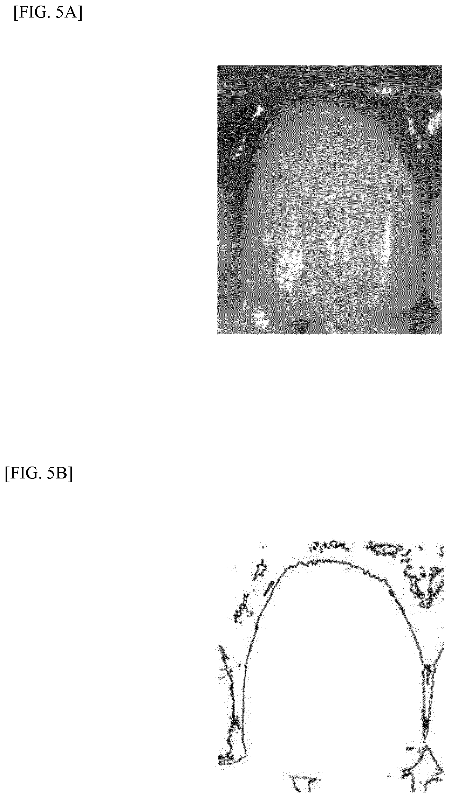

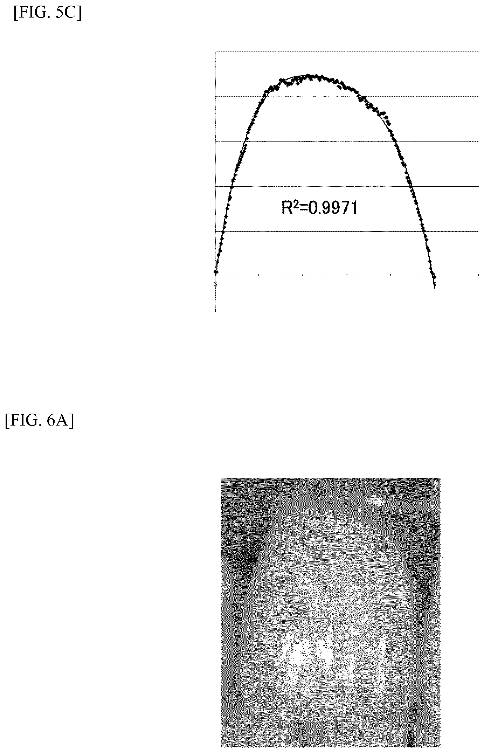

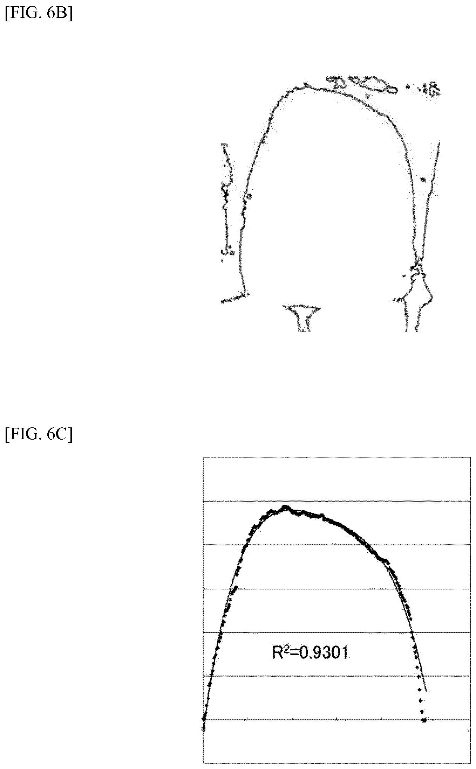

FIGS. 4C, 5C, and 6C are views illustrating the gum diagnosis data obtained by superimposing the fitted curve on the point group data of the border between the tooth and the gum respectively of FIGS. 4B, 5B, and 6B. As described above, the gum diagnosis data is shown as a two-dimensional graph. Note that the fitted curve shown in FIG. 4C is a quadratic polynomial, the fitted curve shown in each of FIGS. 5C and 6C is a quartic polynomial.

The gum diagnosis data may include, for example, a determination coefficient R^2 of the point group data of the border between the tooth and the gum calculated by the correlation calculator 24 and the fitted curve. Thus, the degree of correlation of the fitted curve with the point group data of the border between the tooth and the gum can be obtained as a numerical value.

Referring back to FIG. 3, the display apparatus 30 is an apparatus configured to display the gum diagnosis data generated by the gum diagnosis assisting apparatus 20B. For example, the display apparatus 30 displays the front photographic image of the tooth and the gum of FIGS. 4A, 5A, and 6A and the gum diagnosis data of FIGS. 4C, 5C, and 6C side by side. A dentist watches these images displayed on the display apparatus 30, which enables the dentist to objectively determine the roughness or smoothness of the border between the tooth and the gum, the degree of the deviation from the fitted curve, and the like.

Note that in both of the first embodiment and the second embodiment, original image data acquired by the imaging apparatuses 10A and 10B may be stored in a storage apparatus (not shown), and the gum diagnosis assisting apparatuses 20A and 20B may read any original image data from the storage apparatus and may analyze the original image data. Moreover, the gum diagnosis data generated by the gum diagnosis assisting apparatuses 20A and 20B may be stored in the storage apparatus. Thus, aging of gum of each patient is easily tracked, and thus, a more accurate gum diagnosis based on the aging, a diagnosis for prevention of gingivitis, and the like are possible.

Moreover, results of diagnoses by a dentist in the past may also be stored, and the gum diagnosis assisting apparatuses 20A and 20B may be caused to perform machine learning of tendencies of the diagnoses by the dentist. In this case, it becomes possible to present to the dentist the results of a diagnosis in an example in the past similar to gum diagnosis data which is newly generated. Moreover, based on a large number of diagnoses in the past, the gum diagnosis assisting apparatuses 20A and 20B can propose a gum diagnostic criterion to a dentist.

As described above, the embodiment has been described as an example of the technique in the present invention. For this purpose, attached drawings and detailed description are provided.

Thus, the attached drawings and components described in the detailed description can include not only components essential to solve the problems but also components which are not essential to solve the problems but are shown to illustrate the above-described technique. Thus, the components which are not essential should not directly be recognized as essential based on only that they are included in the attached drawings and/or are described in the detailed description.

Moreover, the above-described embodiment illustrates the technique in the present invention, and therefore, various modifications, replacements, addition, omission, and the like can be performed within the scope of the claims or within the scope of the equivalent thereof.

### Reference Signs List

- 100A, 100B: Gum Diagnosis Assisting System
- 10A, 10B: Imaging Apparatus
- 20A, 20B: Gum Diagnosis Assisting Apparatus
- 21: Sectional Data Extractor
- 22: Contour Extractor
- 23: Curve Fitting Unit
- 24: Correlation Calculator
- 25: Gum Diagnosis Data Generator
- 30: Display Apparatus

## Claims

1. A gum diagnosis assisting apparatus comprising:
a sectional data extractor configured to extract sectional data in a depth direction of a gum marginal portion from three-dimensional point group data of a frontal view of an oral cavity, and
a gum diagnosis data generator configured to process the sectional data into a format suitable to a gum diagnosis to generate gum diagnosis data.

2. The gum diagnosis assisting apparatus according to claim 1, wherein
the gum diagnosis data generator is configured to generate a two-dimensional graph as the gum diagnosis data, where a right-left direction of the frontal view of the oral cavity corresponds to a first axis and a depth direction of the frontal view of the oral cavity corresponds to a second axis.

3. The gum diagnosis assisting apparatus according to claim 1, wherein
the gum diagnosis data generator is configured to generate a two-dimensional graph as the gum diagnosis data, where an up-down direction of the frontal view of the oral cavity corresponds to a first axis and a depth direction of the frontal view of the oral cavity corresponds to a second axis.

4. A gum diagnosis assisting apparatus comprising:
a contour extractor configured to extract a contour of a gum marginal portion from a front photographic image of a tooth and gum to generate contour point group data;
a curve fitting unit configured to perform function fitting on point group data of a border between the tooth and the gum of the contour point group data to derive a fitted curve; and
a gum diagnosis data generator configured to generate gum diagnosis data by superimposing the fitted curve on the point group data of the border between the tooth and the gum.

5. The gum diagnosis assisting apparatus according to claim 3, comprising:
a correlation calculator configured to calculate an index denoting a degree of correlation of the point group data of the border between the tooth and the gum with the fitted curve.

6. A gum diagnosis assisting system comprising:
the gum diagnosis assisting apparatus according to any one of claims 1 to 5;
an imaging apparatus configured to generate original image data of a process target by the gum diagnosis assisting apparatus; and
a display apparatus configured to display gum diagnosis data generated by the gum diagnosis assisting apparatus.
